# DEMANDE DE BREVET EUROPEEN

(11) **EP 3 889 901 A1**
(43) Date de publication de la demande: **06.10.2021**
(21) Numéro de dépôt: 21175754.7
(22) Date de dépôt: 13.05.2004
(51) Int. Cl.: G06T 7/30, A61B 34/30

(54) **DISPOSITIF ET PROCÉDÉ DE RECALAGE EN TEMPS RÉEL DE MOTIFS SUR DES IMAGES, NOTAMMENT POUR LE GUIDAGE PAR LOCALISATION**

(30) Priorité: 22.05.2003 FR 0306176
(62) Demande divisionnaire de: 18165967.3
(71) Demandeur: Intuitive Surgical Operations, Inc., Sunnyvale, CA 94086 (US)
(72) Inventeur: COSTE-MANIERE, Eve, F-06010 Nice (FR); VIEVILLE, Thierry, F-06250 Mougins (FR); MOURGUES, Fabien, F-06400 Cannes (FR)
(74) Mandataire: MacDougall, Alan John Shaw

(57) **Abrégé**

Un dispositif (DS) est dédié au recalage en temps réel de motifs connus, caractéristiques d'une région, sur des images (réelles) de cette région. Ce dispositif (D) comprend, d'une part, une mémoire (21) dans laquelle sont stockés des motifs répresentatifs de portions d'une région choisie et de position et d'orientation connues par rapport à un référentiel commun, et d'autre part, des moyens de traitement (22) chargés, lorsqu'ils reçoivent la désignation d'au moins une portion d'image d'observation de la région choisie, prise sous un angle choisi, et d'au moins un attribut représentatif de cette portion, de déterminer dans la mémoire (21) un motif représentatif de la portion désignée, compte tenu de l'attribut désigné, puis de recaler le motif déterminé sur la portion d'image désignée compte tenu de l'angle choisi.

## Description

L'invention concerne le domaine du traitement de données d'images, et plus précisément le recalage en temps réel de données d'images représentant des motifs connus sur des images d'observation.

Dans un certain nombre de domaines, il est important de savoir précisément, à chaque instant, comment l'on est positionné par rapport à un lieu ou un objet, ou plus généralement une région.

C'est par exemple le cas dans le domaine de la chirurgie, notamment lorsqu'elle met en oeuvre une technique dite "mini-invasive". Dans ce cas, le champ opératoire est observé par une caméra endoscopique introduite dans le corps du patient et délivrant des images sur un ou plusieurs moniteurs (ou dans des lunettes d'observation) en regard duquel (desquelles) se trouve installé un chirurgien. Dans le cas d'une procédure assistée par un robot, le chirurgien pilote à distance les bras manipulateurs du robot dont les extrémités sont également introduites dans le corps du patient. C'est notamment le cas de l'installation Da Vinci ® de la société Intuitive Surgical Inc qui dispose, d'une part, d'un afficheur stéréoscopique, permettant au chirurgien de visualiser en trois dimensions (3D) la région d'intervention, et d'autre part, de manettes de pilotage et de pédales de commande permettant au chirurgien d'adresser des instructions de pilotage et de commande au robot.

Cette technique opératoire est particulièrement intéressante pour le patient dans la mesure où elle est très peu invasive. Mais, elle est particulièrement délicate à mettre en oeuvre du fait qu'elle n'offre au chirurgien, d'une part, qu'une vision partielle et en partie déformée de la région dans laquelle il doit intervenir, en raison de l'utilisation d'une caméra endoscopique, et d'autre part, qu'un espace d'intervention exigu et encombré par les bras manipulateurs du robot et par la caméra endoscopique. En outre, certaines régions, telles que le coeur, étant animées, cela accentue la difficulté de l'intervention.

Afin d'améliorer la situation, il a été proposé de mettre en oeuvre une phase de modélisation pré-opératoire. Dans une telle phase pré-opératoire, on effectue tout d'abord une modélisation tridimensionnelle, et éventuellement temporelle, de la région objet de l'intervention à partir d'images obtenues par imagerie médicale. Dans le cas d'un coeur, on détermine également le réseau coronaire à l'aide de séquences angiographiques, puis on effectue un recalage de ce réseau coronaire sur la surface du coeur obtenue par IRM. On détermine ensuite un modèle anatomique de la partie du corps du patient qui contient la région objet de l'intervention, toujours à partir d'images obtenues par imagerie médicale.

Puis, on détermine, d'une part, les points d'incision optimaux compte tenu du modèle anatomique et de paramètres, tels que la dextérité et l'accessibilité de la région visée, et d'autre part, la configuration optimale des bras du robot, de manière à éviter les collisions et à obtenir une séparation maximale, notamment.

Une fois la phase pré-opératoire terminée, le chirurgien peut procéder à l'intervention. Le patient est alors installé sur la table d'opération, puis on calibre l'endoscope à l'aide d'une grille posée sur la table d'opération et observée selon différents points de vue. Le corps du patient est alors incisé au niveau des points d'incisions optimaux précédemment déterminés. Puis, les bras du robot sont placés dans la configuration optimale précédemment déterminée, et leurs extrémités, tout comme celle de la caméra endoscopique, sont introduites dans le corps du patient par les incisions. L'intervention peut alors commencer.

Malgré la phase pré-opératoire, le chirurgien peut encore avoir des difficultés à localiser avec précision la zone d'intervention. Cela peut notamment survenir lors d'une intervention sur un organe tel que le coeur. Il peut en effet être difficile de localiser l'artère interventriculaire du fait d'une quantité trop importante de graisse à la surface de l'épicarde. Il est également possible de confondre la branche marginale de l'artère circonflexe ou la branche diagonale (particulièrement développée) avec l'artère interventriculaire du fait du fort grossissement de la caméra endoscopique et/ou du faible champ de vision et/ou du faible recul possible et/ou d'un mauvais positionnement de l'ouverture effectuée dans le péricarde.

En complément de ces difficultés à repérer les cibles opératoires, le chirurgien rencontre des difficultés à localiser la caméra endoscopique et par conséquent à estimer l'angle sous lequel il observe la région d'intervention. Enfin, le retour tactile faisant défaut, il n'est pas possible de déterminer la zone à opérer en exerçant sur elle des pressions avec les extrémités des bras du robot.

En résumé, le chirurgien éprouve de réelles difficultés à déterminer la position précise de la zone (ou portion) de la région observée, dans laquelle il doit intervenir, par rapport aux positions connues des extrémités des bras du robot d'intervention.

Pour améliorer encore la situation, il a été proposé d'assister le chirurgien en superposant sur les images endoscopiques de la région observée un motif représentatif d'un élément caractéristique de la portion de région où doit s'effectuer l'intervention, ou d'une portion voisine. Ces motifs sont préalablement extraits des modélisations numériques, effectuées à partir des images obtenues par imagerie médicale. Mais, une telle assistance ne suffit pas, notamment lorsque la région d'intervention comporte plusieurs éléments caractéristiques sensiblement identiques (soit naturellement, soit du fait de l'angle d'observation), comme dans le cas du réseau coronarien.

Des difficultés similaires de détermination précise des positions de lieux ou objets, via des portions d'images réelles, surviennent également dans d'autres domaines techniques, et notamment dans le domaine du guidage urbain.

L'invention a donc pour but de remédier à tout ou partie des inconvénients précités.

Elle propose à cet effet un dispositif dédié au recalage en temps réel de motifs (par exemple tridimensionnels (3D)) connus, caractéristiques d'une région, sur des images (réelles) de cette région.

Ce dispositif se caractérise par le fait qu'il comprend, d'une part, une mémoire dans laquelle sont stockés des motifs représentatifs de portions d'une région choisie et de position et d'orientation connues par rapport à un référentiel commun, et d'autre part, des moyens de traitement chargés, lorsqu'ils reçoivent la désignation d'au moins une portion d'image d'observation de la région choisie, prise sous un angle choisi, et d'au moins un attribut représentatif de cette portion, de déterminer dans la mémoire un motif représentatif de la portion désignée, compte tenu de l'attribut désigné, puis de recaler le motif déterminé sur la portion d'image désignée compte tenu de l'angle choisi.

Le dispositif selon l'invention peut comporter d'autres caractéristiques qui pourront être prises séparément ou en combinaison, et notamment:
* des moyens de traitement capables d'effectuer le recalage par des désignations successives de portions d'images d'observation de la région choisie et/ou d'attributs représentatifs de ces portions,
* une mémoire capable de stocker un modèle tridimensionnel représentatif de la région au sein du référentiel choisi. Dans ce cas, les moyens de traitement sont préférentiellement chargés d'effectuer leur(s) recalage(s) à partir, notamment, du modèle tridimensionnel stocké,
* des moyens de traitement capables de déterminer au moins une équation de mesure à partir de la désignation d'une portion d'image, d'un attribut et d'au moins une hypothèse, et de procéder à la détermination du motif et à son recalage en fonction de l'équation de mesure déterminée. Plusieurs équations de mesure peuvent être déterminées à partir de la désignation de plusieurs portions d'images, d'attributs et d'hypothèses. Dans ce cas, les moyens de traitement procèdent à la détermination du motif et à son recalage en fonction d'une combinaison des équations de mesure déterminées. Le recalage est par exemple de type rigide par minimisation d'un critère choisi prenant en compte les équations de mesure déduites des hypothèses. Par ailleurs, les hypothèses peuvent être transmises aux moyens de traitement par l'utilisateur qui effectue les désignations, ou bien être directement déterminées par les moyens de traitement,
*des moyens de traitement capables de délivrer des données d'images représentatives d'un motif recalé afin qu'on puisse l'observer en même temps que les images d'observation, et de façon superposée, sur la portion d'image désignée correspondante, une fois le recalage effectué,
* une mémoire capable de stocker une table de correspondance entre les motifs et des données d'information qui les représentent. Dans ce cas, les moyens de traitement sont avantageusement chargés de délivrer, chaque fois qu'ils en reçoivent l'ordre, des données d'information représentatives d'un motif recalé,
* des moyens de traitement capables de délivrer sensiblement en même temps, chaque fois qu'ils en reçoivent l'ordre, des données d'images représentatives d'un motif recalé et les données d'information représentatives de ce motif.

L'invention concerne en outre une installation de guidage par localisation comprenant, d'une première part, des moyens d'observation pouvant délivrer des images d'une région choisie sous un angle choisi, d'une deuxième part, un afficheur (par exemple un ou plusieurs moniteurs d'ordinateur ou des lunettes d'observation) permettant d'afficher ces images, d'une troisième part, une interface homme/machine permettant à un utilisateur de désigner au moins une portion de la région représentée par les images affichées et au moins un attribut représentatif de cette portion, et d'une quatrième part, un dispositif de recalage du type de celui présenté ci-avant, alimenté en portion(s) et attribut(s) désignés par l'interface homme/machine et délivrant des données d'images représentatives d'un motif recalé de sorte que ce dernier soit superposé par l'afficheur sur la portion désignée affichée, une fois le recalage effectué.

Les moyens d'observation peuvent comprendre des moyens d'acquisition, par exemple de type endoscopique, dont la position est connue à chaque instant par rapport à un référentiel de calibration, à partir duquel est définie la position de la région observée, et capable de délivrer des images d'observation à l'afficheur.

Lorsque l'installation ne sert qu'au guidage, par exemple urbain, elle comprend préférentiellement des moyens de contrôle chargés, lorsqu'ils reçoivent une requête désignant un motif de la région observée, d'ordonner au dispositif de recalage de déterminer des données de position représentatives de la position de ce motif par rapport au référentiel de calibration, compte tenu du recalage, puis de déterminer des instructions de pilotage destinées à guider l'utilisateur vers la portion qui correspond à ce motif.

Lorsque l'installation sert aux interventions chirurgicales, elle peut comprendre un robot d'intervention comportant des bras dont les positions respectives par rapport au référentiel de calibration sont connues à chaque instant et qui peuvent être pilotés à distance par des instructions fournies par un utilisateur via l'interface homme/machine. Mais, elle comprend également des moyens de contrôle couplés au dispositif de recalage et à l'interface homme/machine, et chargés, lorsqu' ils reçoivent une requête désignant un motif de la région observée, d'une part, d'ordonner au dispositif de recalage de déterminer des données de position représentatives de la position de ce motif par rapport au référentiel de calibration compte tenu du recalage, et d'autre part, de déterminer des instructions de pilotage destinées à déplacer les bras du robot au voisinage de la portion de région correspondant au motif désigné.

L'invention concerne également un procédé dédié au recalage en temps réel de motifs (par exemple 3D) connus, caractéristiques d'une région, sur des images de cette région, et consistant à :
* stocker dans une mémoire des motifs représentatifs de portions d'une région choisie et de position et d'orientation connues par rapport à un référentiel commun,
* observer sous un angle choisi la région choisie et délivrer en temps réel des images d'observation de cette région,
* désigner au moins une portion d'image d'observation de la région choisie et au moins un attribut représentatif de cette portion,
* déterminer dans la mémoire un motif représentatif de la portion désignée, compte tenu de l'attribut désigné, et
* recaler le motif sur la portion d'image désignée compte tenu de l'angle choisi.

Le procédé selon l'invention peut comporter d'autres caractéristiques qui pourront être prises séparément ou en combinaison, et notamment:
* on peut effectuer le recalage par des désignations successives de portions d'images d'observation de la région choisie et/ou d'attributs représentatifs de ces portions,
* on peut stocker dans la mémoire un modèle 3D représentatif de la région dans le référentiel choisi, et effectuer chaque recalage au moins à partir de ce modèle 3D,
* on peut effectuer un recalage de type rigide, par minimisation d'un critère choisi. Dans ce cas, on détermine préférentiellement au moins une équation de mesure à partir de la désignation d'une portion d'image, d'un attribut et d'au moins une hypothèse, et l'on procède à la détermination de motif et au recalage rigide en fonction de l'équation de mesure déterminée. On peut déterminer plusieurs équations de mesure à partir de la désignation de plusieurs portions d'images, d'attributs et d'hypothèses, constituant les contraintes, et procéder à la détermination du motif et à son recalage rigide en fonction d'une combinaison des équations de mesure déterminées. Certaines au moins des hypothèses peuvent être transmises par l'utilisateur effectuant les désignations, par exemple sous la forme d'attributs,

* on peut délivrer des données d'images représentatives d'un motif recalé afin qu'il soit observé en même temps que les images d'observation, et de façon superposée, sur la portion d'image désignée correspondante,
* on peut stocker dans la mémoire une table de correspondance entre les motifs et des données d'information représentatives de ces motifs, et délivrer sur requête des données d'information représentatives d'un motif,
* on peut afficher les données d'images représentatives d'un motif recalé,
* on peut procéder aux désignations via une interface homme/machine,
* lorsque l'on reçoit une requête d'un utilisateur désignant un motif contenu dans la région observée, on peut déterminer des données de position représentatives de la position de ce motif par rapport au référentiel de calibration (à partir duquel est définie la position de la région observée), compte tenu du recalage, puis déterminer des instructions de pilotage destinées à guider l'utilisateur vers la portion qui correspond audit motif désigné,
* on peut prévoir un robot d'intervention comportant des bras dont les positions respectives par rapport au référentiel choisi sont connues à chaque instant et peuvent être pilotés à distance par des instructions fournies par un utilisateur via l'interface homme/machine, et lorsque l'on reçoit une requête d'utilisateur désignant un motif de la région choisie, on peut déterminer des données de position représentatives de la position du motif par rapport au référentiel de calibration, compte tenu du recalage, puis déterminer des instructions de pilotage destinées à déplacer les bras du robot au voisinage de la portion de région correspondant au motif désigné.

D'autres caractéristiques et avantages de l'invention apparaîtront à l'examen de la description détaillée ci-après, et des dessins annexés, sur lesquels :
- la figure 1 illustre de façon schématique un exemple de réalisation d'une installation selon l'invention, adapté à une application dans le domaine de la téléchirurgie mini-invasive,
- la figure 2 illustre de façon schématique un motif 3D représentatif d'une artère coronaire,
- la figure 3 illustre de façon schématique un motif 3D représentatif de trois artères coronaires voisines,
- la figure 4 illustre de façon schématique un motif 3D représentatif d'une artère coronaire et de trois bifurcations,
- la figure 5 illustre de façon schématique un motif 3D représentatif d'un secteur angulaire au niveau d'une bifurcation entre deux artères coronaires, et
- la figure 6 est une image d'un coeur sur laquelle a été superposé un modèle 3D, après recalage.

Les dessins annexés pourront non seulement servir à compléter l'invention, mais aussi contribuer à sa définition, le cas échéant.

L'invention concerne d'une manière générale le recalage en temps réel de données d'images représentant des motifs connus, par exemple tridimensionnels (3D), caractéristiques d'une région, sur des images d'observation de cette région. Mais, elle concerne également les installations de guidage par localisation utilisant un tel recalage, comme par exemple les installations de guidage urbain et de téléchirurgie, en particulier de type "mini-invasif'.

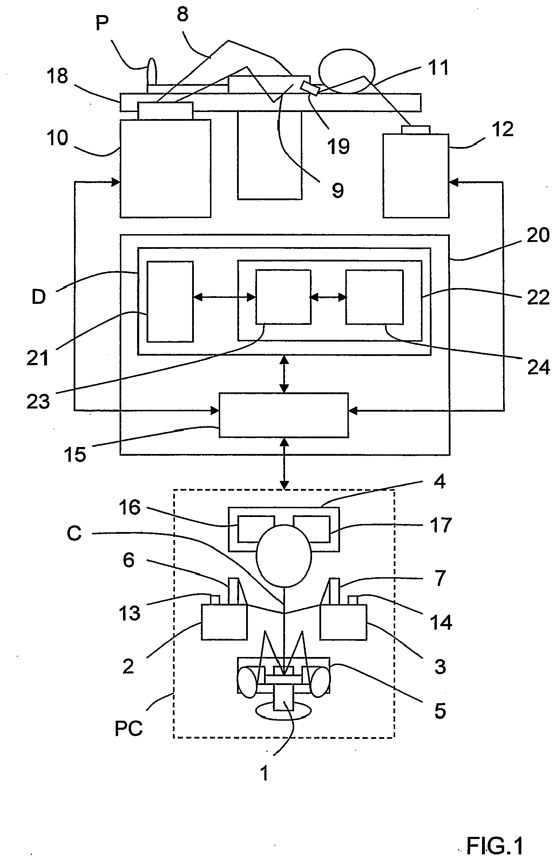

On se réfère tout d'abord à la figure 1 pour décrire un exemple de réalisation, non limitatif, d'une installation selon l'invention adaptée à la téléchirurgie mini-invasive.

L'installation de téléchirurgie illustrée est par exemple constituée àpartir de l'installation Da Vinci ® de la société Intuitive Surgical Inc. Elle comprend schématiquement un poste de commande PC comportant une chaise 1 permettant à un chirurgien C de s'installer devant une console équipée d'un clavier de commande (non représenté), d'une première commande manuelle 2 pour la main gauche, d'une seconde commande manuelle 3 pour la main droite, d'un dispositif d'affichage 4, ici de type stéréoscopique, et d'un ensemble de pédales de commande 5.

Chaque commande manuelle 2, 3 comporte par exemple un levier de pilotage 6, 7 (de type "joystick"), destiné à piloter l'un des bras manipulateurs 8, 9 d'un robot 10, sur lequel on reviendra plus loin, ainsi que le bras manipulateur 11 d'une caméra stéréoscopique 12, sur laquelle on reviendra également plus loin, et une ou plusieurs touches 13, 14 de commande (de type sensitif, ou bouton-poussoir, ou encore "souris").

L'ensemble de pédales 5 comporte par exemple une pédale permettant d'associer une commande manuelle 2, 3 au pilotage du robot d'intervention 10, une pédale permettant d'associer une commande manuelle 2, 3 au pilotage de la caméra 12, et une pédale permettant d'associer une commande manuelle 2, 3 au pilotage d'un module de contrôle 15 de l'installation, sur lequel on reviendra plus loin.

Le clavier de commande, les commandes manuelles 2 et 3, et l'ensemble de pédales de commande 5 constituent une interface homme/machine.

Le dispositif d'affichage 4 comprend, ici, un premier écran 16, pour l'affichage d'images bidimensionnelles (2D) réelles délivrées par une première voie de la caméra 12 et destinées à l'oeil gauche du chirurgien C, et un second écran 17, pour l'affichage d'images bidimensionnelles (2D) réelles délivrées par une seconde voie de la caméra 12 et destinées à l'oeil droit du chirurgien C.

Le robot d'intervention 10 est destiné à être placé à proximité de la table d'opération 18, sur laquelle est installé le patient P devant être opéré de façon mini-invasive. Il comprend généralement deux bras manipulateurs 8 et 9 munis d'extrémités adaptées à l'opération et destinées à être introduites dans le corps du patient P via des incisions.

La caméra stéréoscopique 12 comporte un bras manipulateur 11 dont l'extrémité supporte deux fibres optiques endoscopiques 19 définissant deux voies d'acquisition d'images.

Le robot d'intervention 10 et la caméra endoscopique 12 peuvent être réunis pour constituer un "maître robot".

L'installation comporte en outre un ensemble de contrôle 20, par exemple agencé sous la forme d'une station de travail, comportant le module de contrôle 15 et un dispositif de recalage selon l'invention D, sur lequel on reviendra plus loin. Le module de contrôle 15 est couplé à la console du poste de commande PC, au robot d'intervention 10, à la caméra stéréoscopique 12 et au dispositif de recalage D.

Le dispositif de recalage D, selon l'invention, est destiné, d'une manière générale, à recaler en temps réel des motifs connus, qui sont caractéristiques d'une région (ici dans laquelle doit s'effectuer une opération), sur des images (réelles) de cette région. Dans ce qui suit, on considère que les motifs sont tridimensionnels (3D), mais ils pourraient être bidimensionnels (2D), au moins pour certains d'entre eux.

Ce dispositifD comprend tout d'abord une mémoire 21, stockant des motifs tridimensionnels (3D) représentatifs de portions caractéristiques de la région dans laquelle doit avoir lieu l'intervention, et de position et d'orientation connues par rapport à un référentiel commun (ou référentiel pré-opératoire). Le dispositif D comprend également un module de traitement 22 couplé à la mémoire 21 et chargé, quand il reçoit les désignations, d'une part, d'au moins une portion d'image d'observation de la région d'intervention, prises sous un angle choisi par la caméra endoscopique 12 et délivrées par le module de contrôle 15, et d'autre part, d'au moins un attribut représentatif de la portion désignée, de déterminer dans la mémoire 21 un motif 3D qui est représentatif de cette portion désignée, compte tenu de l'attribut désigné et de l'angle de prise de vue choisi, puis de recaler le motif 3D déterminé sur la portion d'image désignée.

Il est important de noter qu'un ensemble de motifs 3D peut constituer un modèle 3D. Par conséquent, le recalage peut porter non seulement sur un motif 3D, mais également sur un modèle 3D.

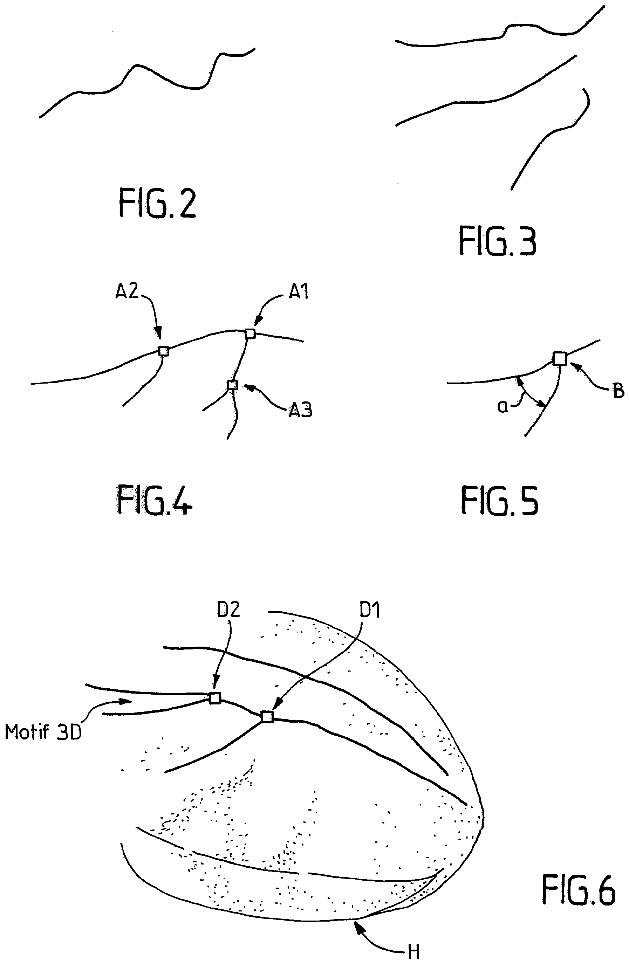

Par exemple dans le cas du coeur, un modèle 3D peut être représentatif de l'arbre coronarien, ce modèle 3D étant alors constitué d'une multiplicité de motifs 3D représentatifs de structures caractéristiques de l'arbre coronarien, comme par exemple des artères, des jonctions et des bifurcations. En fait, dans le cas d'un arbre coronarien, on définit deux types de structures. Un premier type regroupe des courbes représentant les artères, tandis qu'un second type regroupe des éléments caractéristiques, comme par exemple des jonctions ou des bifurcations. Ces différents types sont illustrés sur les figures 2 à 5.

Plus précisément, la figure 2 illustre un motif représentatif d'une artère coronaire, la figure 3 illustre un motif représentatif d'une configuration de trois artères coronaires, la figure 4 illustre un motif représentatif d'une configuration de trois bifurcations sur une artère coronaire, et la figure 5 illustre un motif représentatif d'un angle α caractéristique d'une bifurcation entre deux artères coronaires.

Par exemple, une bifurcation est définie, d'une première part, par un index (entier identifiant la bifurcation), d'une deuxième part, par deux index (Art1 et Art2 qui identifient les deux artères concernées), et d'une troisième part, par un point (triplet de coordonnées de la bifurcation dans le référentiel pré-opératoire). De même, une artère est définie, d'une part, par un index (entier identifiant l'artère), et d'autre part, par un ensemble de paramètres d'une B-spline composant la ligne centrale de l'artère (quadruplet (xi, yi, zi, ui) dans lequel (xi, yi, zi) est un triplet définissant chaque point de contrôle de l'artère dans le référentiel pré-opératoire, et ui désigne un noeud de la B-spline dont la valeur est comprise entre 0 et 1).

L'obtention des motifs 3D ne faisant pas l'objet de l'invention, elle ne sera pas décrite ici en détail. Une description précise d'un mode d'obtention est par exemple donnée dans l'article de Ève Coste-Manière et al "Optimal Planning of Robotically Assisted Heart Surgery : Transfert Precision in the Operating Room", B. Siciliano and P. Dario (Eds.) : Expérimental Robotics VIII, STAR 5, pp. 424-434, 2003, ou dans le document de Fabien Mourgues et al "3D+t Modeling of coronary artery tree from standard non simultaneous angiograms" : Proc. of MICCAI, Volume 2208 of LNCS, Spinger (2001), 1320-1322.

Il est simplement rappelé que l'obtention de motifs 3D d'une région d'intervention, comme par exemple un organe tel que le coeur, nécessite tout d'abord une modélisation tridimensionnelle, et éventuellement temporelle (3D + t), de la région d'intervention à partir d'images obtenues par imagerie médicale (IRM, scanner, etc). Par exemple, dans le cas d'un coeur, on peut obtenir par IRM son modèle volumique (3D) à un instant donné du cycle cardiaque. Pour obtenir un modèle 3D + t complet, on anime le modèle volumique (3D) à partir d'un modèle 3D + t du réseau coronarien du coeur modélisé, obtenu à partir de séquences d'images angiographiques (rayons X), également appelées coronarographies, prises sous des angles différents et durant plusieurs cycles, et synchronisées par rapport à un électrocardiogramme (ECG).

Dans l'exemple précité d'un modèle 3D d'arbre coronarien, les motifs 3D sont donc des fractions (ou portions) caractéristiques de l'arbre (ou réseau) coronarien 3D dont les positions sont connues par rapport au modèle volumique (3D) du coeur du patient P.

Le référentiel pré-opératoire par rapport auquel sont définies les positions des motifs 3D et du coeur est habituellement celui de l'enveloppe externe du patient P. C'est en effet par rapport à cette enveloppe externe que peuvent être calibrés le robot d'intervention 10 et la caméra endoscopique 12. De plus, le fait de rapporter la position du coeur, et donc de son réseau coronarien, par rapport à l'enveloppe externe permet de recaler le patient par rapport au robot en salle d'opération.

On détermine également un modèle anatomique 3D du corps du patient, au moins dans une vaste partie contenant la région objet de l'intervention, toujours à partir d'images obtenues par imagerie médicale.

Les positions, orientations et configurations des motifs (et modèles) 3D sont donc stockées dans la mémoire 21 par rapport à un référentiel commun défini à partir de repères placés sur l'enveloppe externe du patient P.

Préférentiellement, on stocke également dans la mémoire 21 le modèle 3D du coeur du patient P, ainsi que son modèle anatomique 3D.

L'invention n'ayant pas pour objet la phase pré-opératoire de planification de l'opération chirurgicale, celle-ci ne sera pas décrite ici. Par conséquent, on décrit dans ce qui suit la mise en oeuvre du dispositif D selon l'invention, au sein d'une installation selon l'invention, dans la phase opératoire.

Il est simplement rappelé que la phase de planification pré-opératoire, qui n'est qu'optionnelle, a notamment pour objet de déterminer les trois points d'incision optimaux, qui vont permettre d'introduire les extrémités des bras manipulateurs 8, 9 et 11 du robot d'intervention 10 et de la caméra endoscopique 12, compte tenu du modèle anatomique 3D du corps du patient P et de paramètres, tels que la dextérité et l'accessibilité de la région visée. Elle consiste également à déterminer la configuration optimale des bras manipulateurs 8 et 9 du robot d'intervention 10 afin d'éviter les collisions et d'obtenir une séparation maximale.

Le dispositif D intervient au sein de l'installation une fois que le bras 11 et la caméra endoscopique 12 (éventuellement endoscopique) ont été calibrés. Il s'agit, ici, de déterminer précisément les paramètres optiques de la caméra et sa position dans le référentiel du bras 11. Un exemple de méthode de calibration endoscopique est décrit dans le document de F. Mourgues et al "Flexible calibration of actuated stereoscopic endoscope for overlay in robot assisted surgery", Proc. of MICCAI, Volume 2488 of LNCS, Spinger (2002), 25-34. Les données de calibration sont préférentiellement stockées dans une mémoire du module de contrôle 15.

On réalise ensuite des incisions dans le corps du patient P, au niveau des points d'incisions optimaux déterminés pendant la phase de planification pré-opératoire. Les bras manipulateurs 8 et 9 du robot d'intervention 10 et celui 11 de la caméra endoscopique sont alors placés dans la configuration optimale, également déterminée pendant la phase de planification pré-opératoire, et leurs extrémités respectives sont introduites dans le corps du patient P par les incisions. Il est rappelé que la détermination des points d'incision optimaux est une option préférée, mais non obligatoire, les positionnements des bras du robot et de l'endoscope pouvant être déterminés de façon empirique par le chirurgien.

Les deux voies de la caméra endoscopique 12 délivrent leurs séquences d'images 2D respectives au module de contrôle 15, qui les transmet au dispositif d'affichage 4 de sorte qu'elles s'affichent sur les écrans 16 et 17. Le chirurgien C peut alors observer la région dans laquelle est implanté le coeur H objet de l'intervention, sous l'angle d'observation de la caméra endoscopique 12. La figure 6 est une image de coeur H du type de celles qui sont observées par le chirurgien C sur les écrans 16 et 17.

La région d'intervention étant connue, on peut dès le début de l'intervention proposer une première superposition du modèle 3D (ici de l'arbre coronarien) sur les images d'observation affichées. Cela peut se faire manuellement ou par un recalage externe du patient, installé sur la table d'opération, avec son modèle pré-opératoire. Le recalage externe consiste tout d'abord à pointer avec l'extrémité du robot plusieurs marqueurs radio-opaques préalablement collés sur le thorax du patient, et précédemment segmentés dans les images scanners. Puis, on calcule la transformation rigide entre les marqueurs pointés et les marqueurs segmentés. Cette transformation rigide initiale permet de passer du référentiel pré-opératoire (référentiel dans lequel sont représentés les motifs du modèle 3D à recaler) au référentiel de la base du robot, et au référentiel de la caméra endoscopique (en utilisant alors la calibration de l'endoscope). Cette technique de recalage externe est notamment décrite dans le document de E. Coste-Manière et al "Optimal planning of robotically assisted heart surgery : Transfer precision in the operating room, B. Siciliano et P. Dario, eds, Springer Tracts In Advanced Robotics, Expérimental Robotics VIII, Volume 5, Springer (2002), 424-434.

Une fois le recalage externe éventuellement effectué, on repère précisément la zone dans laquelle doit avoir lieu l'intervention. Pour ce faire, le chirurgien C désigne au moins une portion des images affichées sur ses écrans 16 et 17 et au moins un attribut représentatif de chaque portion d'image à l'aide de son interface homme/machine. La désignation d'une portion s'effectue par exemple en sélectionnant la portion sur l'image, avec une souris. La désignation d'un attribut s'effectue soit par une commande vocale, soit par sélection dans une liste affichée sur les écrans 16 et 17 ou sur un écran auxiliaire. Dans l'exemple illustré sur la figure 4, les carrés A1 à A3 matérialisent les endroits sur lesquels le chirurgien C "clique" avec sa souris pour désigner trois portions de la région qui lui paraissent significatives. De même, dans l'exemple illustré sur la figure 5, le carré B matérialise l'endroit sur lequel le chirurgien C "clique" avec sa souris pour désigner la portion de la région qui lui parait significative.

Les attributs sont des informations types (ou classes) qui décrivent chacune une caractéristique locale connue ou une configuration locale connue, ou d'une manière générale toute information permettant au module de traitement 22 du dispositif D de déterminer dans la mémoire 21 le motif 3D correspondant.

Une fois en possession des désignations fournies par le chirurgien C, le dispositif D les transmet à son module de traitement 22, afin qu'il détermine dans la mémoire 21 un motif 3D qui lui paraît représentatif de la ou des portions désignées, compte tenu de (ou des) l'attribut(s) désigné(s).

Cette détermination est assurée par un module d'extraction 23 couplé à la mémoire 21. Puis, le module de traitement 22 doit effectuer le recalage de ce motif (et éventuellement de l'ensemble du modèle 3D dont il fait partie). Il s'agit ici de déterminer comment orienter et positionner le motif pour qu'il puisse être superposé à la portion désignée par le chirurgien, compte tenu de l'angle sous lequel la région d'intervention est observée par la caméra endoscopique.

Le recalage est préférentiellement effectué par un module de recalage 24 du module de traitement 22. Par ailleurs, ce recalage est préférentiellement rigide, par minimisation d'un critère construit à partir des équations de mesure. Les équations de mesure sont déduites ici des hypothèses fournies par le chirurgien, lorsqu'il pense reconnaître une portion, comme par exemple une artère ou une bifurcation entre artères connues, ou bien directement déterminées par le module de recalage 24. Bien entendu, un autre type de recalage pourrait être envisagé, notamment un recalage affine.

Par exemple, le recalage rigide consiste à déterminer au moins une équation de mesure à partir de la désignation d'une portion d'image, d'un attribut et d'au moins une hypothèse sur l'identité du motif déterminé par le module d'extraction 23. Ce recalage peut également prendre en compte le modèle 3D du coeur lorsque celui-ci est stocké dans la mémoire 21.

Plusieurs équations de mesure peuvent être déterminées à partir de la désignation de plusieurs portions d'images, de plusieurs attributs et d'une ou plusieurs hypothèses, qui constituent autant de contraintes. Dans ce cas, le module de recalage 24 génère plusieurs jeux d'équations correspondants aux différentes hypothèses possibles et optimise ensuite les paramètres de recalage en minimisant un critère. Il classe ensuite les recalages obtenus en fonction de leur pertinence et sélectionne le meilleur recalage. La méthode d'estimation de paramètres est notamment décrite dans le document de T. Vieville et al "Implementing a multi-model estimation method", The International Journal of Computer Vision, 44 (2001), 41-64.

Mais toute autre technique connue d'estimation de paramètres peut être utilisée.

Une fois le recalage rigide du motif 3D effectué, il est possible de recaler l'intégralité du modèle 3D dont fait partie ledit motif. Ainsi, c'est le modèle 3D entier, vue sous l'angle d'observation de la caméra endoscopique, et non seulement le motif 3D déterminé et recalé, qui peut être superposé à l'image affichée ou observée. Bien entendu, certaines portions du modèle 3D peuvent ne pas être visibles du fait de l'angle d'observation.

Le module de traitement 22 peut alors transmettre au module de contrôle 15 les données de position du motif 3D recalé (ou de l'ensemble du modèle 3D dont il fait partie), dans le référentiel (de calibration) de l'image affichée (ou observée, lorsque le chirurgien est équipé de lunettes d'observation), et les données d'image qui définissent ce motif (ou modèle) 3D, afin qu'il ordonne au dispositif d'affichage 4 son affichage de façon superposée sur les images d'observation délivrées par la caméra endoscopique 12 (ou observées dans les lunettes).

Une superposition d'un vaste motif 3D sur une image de coeur est illustrée sur la figure 6. Les carrés D1 et D2 y matérialisent les endroits sur lesquels le chirurgien C a cliqué avec sa souris pour désigner deux portions de la région d'intervention qui lui paraissaient significatives, et la fraction du réseau coronaire superposée à l'image du coeur H y matérialise le motif 3D recalé par le dispositif D.

Grâce à cette superposition, le chirurgien sait immédiatement comment il est positionné par rapport à la zone qu'il doit opérer.

Dans certaines situations, les désignations faites par le chirurgien C peuvent ne pas permettre au module d'extraction 23 de déterminer le motif 3D qui correspond à la portion sélectionnée, ou au module de recalage 24 d'effectuer un recalage approprié, entraînant alors une mauvaise superposition du motif (ou modèle) 3D sur l'image affichée. Par conséquent, le module de traitement 22 peut être agencé de manière à déterminer précisément la position de la région observée dans le référentiel par des recalages successifs reposant chacun sur l'extraction d'un nouveau motif 3D consécutivement à la désignation d'au moins une autre portion d'images d'observation et d'au moins un attribut représentatif de cette autre portion.

Dans cette situation, deux cas peuvent être envisagés. Dans un premier cas, le motif 3D déterminé par le module d'extraction 23 n'est pas superposé sur l'image de la région observée à la portion sélectionnée par le chirurgien C ou bien le motif 3D recalé est superposable, mais il ne correspond pas à la structure observée par le chirurgien sur la portion sélectionnée. Le chirurgien doit alors effectuer une nouvelle désignation pour faire converger l'opération de recalage. Dans un deuxième cas, c'est le module de contrôle 15 qui s'aperçoit automatiquement de l'erreur et qui adresse un message au chirurgien, requérant de sa part de nouvelles, désignations.

A réception de ces nouvelles désignations, le dispositif de recalage D réitère les traitements présentés ci-avant en prenant en compte les nouvelles et les anciennes désignations. Il peut notamment, lorsqu'il est agencé à cet effet, calculer de nouvelles hypothèses et équations de mesure.

On peut par ailleurs envisager de stocker dans la mémoire 21 une table de correspondance entre les motifs 3D et des données d'information qui les représentent. Il peut s'agir par exemple du nom du motif, comme par exemple le nom d'une artère et/ou de ses éventuelles ramifications, ou bien des coordonnées d'un point cible, ou encore de données opératoires enregistrées lors de la phase de planification, comme par exemple le repérage d'une sténose ou d'une zone de calcification.

Dans ce cas, le module de traitement 22 peut être configuré de manière à délivrer les données d'information associées à un motif 3D (recalé ou non) lorsqu'il en reçoit l'ordre du module de contrôle 15 (par exemple en cas de demande du chirurgien C). Mais, on peut également envisager un fonctionnement automatique dans lequel le module de traitement 22, chaque fois qu'il a recalé un motif 3D et qu'il s'apprête à en délivrer les données de position et d'image au module de contrôle 15, extrait de la mémoire 21 les données d'information associées de manière à les communiquer sensiblement simultanément.

Le module de contrôle 15 peut être également conçu, chaque fois qu'il reçoit une requête désignant un motif 3D de la région observée, de manière à, d'une part, ordonner au dispositif de recalage D de déterminer des données de position représentatives de la position de ce motif 3D par rapport au référentiel de calibration, compte tenu du recalage, et d'autre part, déterminer des instructions de pilotage destinées à déplacer les bras manipulateurs 8 et 9 du robot d'intervention 10 au voisinage de la portion de région qui correspond au motif 3D désigné.

L'installation présentée ci-avant peut être utilisée pour d'autres types d'opération, comme par exemple la chirurgie ouverte hépatique ou mammaire. D'une manière générale, l'installation selon l'invention permet le guidage d'un utilisateur dans l'accomplissement de sa tâche dans un environnement où il ne possède qu'une vision partielle et/ou déformée, et/ou dans un environnement difficile.

Par ailleurs, comme indiqué précédemment, le dispositif de recalage D, selon l'invention, peut être utilisé dans d'autre applications que celles présentées ci-avant. Il peut notamment être utilisé dans des installations dédiées au seul guidage par localisation (sans intervention d'un robot télé-opéré), et en particulier dans les zones urbaines.

On peut en effet envisager une installation embarquée dans un véhicule automobile, et comprenant une ou plusieurs caméras, délivrant à un module de contrôle des images réelles de l'environnement de sorte qu'elles soient affichées sur des écrans installés dans l'habitacle, et un dispositif de recalage D couplé audit module de contrôle. L'installation peut être également couplée à un dispositif embarqué de guidage par satellites, de type GPS.

Dans cette application, le dispositif de recalage D stocke dans sa mémoire des motifs représentatifs de l'environnement (ou région) dans lequel peut évoluer le véhicule et déterminés à partir d'enregistrements numériques précédemment réalisés. Ces motifs, dont les positions sont définies dans un repère choisi, sont par exemple des façades d'immeubles, ou des bâtiments ou sites remarquables, ou encore des statues ou oeuvres d'art. La mémoire peut également comporter un modèle volumique de l'environnement, ainsi qu'une table de correspondance entre les motifs et des données d'information sur ces motifs.

Une fois les caméras calibrées par rapport à un référentiel de calibration, l'installation peut être utilisée.

Le module de contrôle est ici chargé, lorsqu'il reçoit d'un passager du véhicule une requête désignant un motif de la région observée (qu'il a sélectionné dans la liste des motifs stockés à l'aide d'une souris ou en exerçant une pression sur un écran d'affichage tactile), d'ordonner au dispositif de recalage D de déterminer des données de position représentatives de la position de ce motif par rapport au référentiel de calibration, compte tenu du recalage induit par les décalages entre le repère choisi et le repère de calibration, puis de déterminer des instructions de pilotage destinées à guider le conducteur du véhicule vers la portion de la région qui correspond à ce motif. Comme indiqué ci-dessus, le module de contrôle peut éventuellement s'appuyer sur les données de position du véhicule, délivrées par le dispositif GPS, pour déterminer les instructions de pilotage. Une fois le véhicule arrivé sur place, le motif peut être éventuellement superposé à l'image réelle de la portion de région, et des données d'information associées audit motif peuvent être délivrées au passager.

Mais, on peut également envisager un fonctionnement automatique dans lequel le dispositif de recalage D reçoit d'un passager du véhicule les désignations d'au moins une portion de région observée et affichée (qu'il a sélectionnée à l'aide d'une souris ou en exerçant une pression sur l'écran d'affichage tactile) et d'au moins un attribut représentatif de cette portion, comme par exemple la nature d'un bâtiment (maison, école, mairie, musée, église) ou d'un lieu (jardin, parc, place) ou d'un objet (statue, sculpture, oeuvre d'art), et détermine dans sa mémoire le motif qui correspond au mieux à cette portion désignée, compte tenu de l'attribut désigné et de l'angle de prise de vue choisi. Puis, il recale le motif déterminé sur la portion d'image désignée. Il en déduit alors les données de position du motif recalé, dans le référentiel de l'image affichée (référentiel de calibration), et les transmet, accompagnées des données d'image qui définissent ce motif et d'éventuelles données d'information associées, au module de contrôle qui ordonne leur affichage sur les écrans, de façon superposée, sur les images d'observation délivrées par les caméras. Ce fonctionnement est donc similaire à celui décrit précédemment dans l'application chirurgicale.

On peut également envisager une variante d'installation adaptée au guidage par localisation d'utilisateurs circulant à pied dans un environnement, par exemple urbain. Dans ce cas, le dispositif selon l'invention est avantageusement implanté dans un équipement de communication, comme par exemple un téléphone mobile, équipé d'une fonction de localisation, par exemple par triangulation ou par GPS, et d'une caméra, ainsi qu'éventuellement d'une centrale inertielle.

Le module de traitement 22 du dispositif de recalage D et le module de contrôle 15 des installations peuvent être réalisés sous la forme de circuits électroniques, de modules logiciels (ou informatiques), ou d'une combinaison de modules logiciels et de circuits électroniques.

L'invention concerne également un procédé dédié au recalage en temps réel de motifs connus, caractéristiques d'une région, sur des images de cette région.

Celui-ci peut être mis en œuvre à l'aide du dispositif de recalage et des installations présentés ci-avant. Les fonctions et sous-fonctions principales et optionnelles assurées par les étapes de ce procédé étant sensiblement identiques à celles assurées par les différents moyens constituant le dispositif et les installations, seules seront résumées ci-après les étapes mettant en œuvre les fonctions principales du procédé selon l'invention.

Ce procédé se caractérise par le fait qu'il consiste à :
* stocker dans une mémoire 21 des motifs représentatifs de portions d'une région choisie et de position et d'orientation connues par rapport à un référentiel commun,
* observer sous un angle choisi la région choisie et délivrer en temps réel des images d'observation de cette région,
* désigner au moins une portion d'image d'observation de la région choisie et au moins un attribut représentatif de cette portion,
* déterminer dans la mémoire un motif représentatif de la portion désignée, compte tenu de l'attribut désigné, et
* recaler le motif sur la portion d'image désignée compte tenu de l'angle choisi.

L'invention ne se limite pas aux modes de réalisation de dispositif, installations et procédé décrits ci-avant, seulement à titre d'exemple, mais elle englobe toutes les variantes que pourra envisager l'homme de l'art dans le cadre des revendications ci-après.

La présente demande comporte également les clauses numérotées suivantes.

### Clauses

1. Dispositif de recalage en temps réel de motifs sur des images d'une région, caractérisé en ce qu'il comprend une mémoire propre à stocker une multiplicité de motifs, représentatifs de portions d'une région choisie et de position et d'orientation connues par rapport à un référentiel commun, des moyens de traitement agencés, à partir de la désignation d'au moins une portion d'image d'observation de ladite région choisie, prise sous un angle choisi, et d'au moins un attribut représentatif de ladite portion, pour déterminer dans ladite mémoire un motif représentatif de ladite portion désignée, compte tenu dudit attribut désigné, puis pour recaler ledit motif déterminé sur ladite portion d'image désignée compte tenu dudit angle choisi.
2. Dispositif selon la clause 1, caractérisé en ce que lesdits moyens de traitement sont agencés pour effectuer ledit recalage par des désignations successives de portions d'images d'observation de ladite région choisie et/ou d'attributs représentatifs desdites portions.
3. Dispositif selon l'une des clauses 1 et 2, caractérisé en ce que lesdits motifs sont tridimensionnels.
4. Dispositif selon la clause 3, caractérisé en ce que ladite mémoire est propre à stocker un modèle tridimensionnel représentatif de ladite région au sein dudit référentiel commun, et en ce que lesdits moyens de traitement sont agencés pour effectuer ledit recalage au moins à partir dudit modèle tridimensionnel stocké.
5. Dispositif selon l'une des clauses 1 à 4, caractérisé en ce que lesdits moyens de traitement sont agencés pour effectuer un recalage de type rigide, par minimisation d'un critère choisi.
6. Dispositif selon la clause 5, caractérisé en ce que lesdits moyens de traitement sont agencés pour déterminer au moins une équation de mesure à partir de la désignation d'une portion d'image, d'un attribut et d'au moins une hypothèse, et pour procéder à ladite détermination du motif et à son recalage rigide en fonction de l'équation de mesure déterminée.
7. Dispositif selon la clause 5, caractérisé en ce que lesdits moyens de traitement sont agencés pour déterminer plusieurs équations de mesure à partir de la désignation de plusieurs portions d'images, d'attributs et d'hypothèses, et pour procéder à ladite détermination du motif et à son recalage rigide en fonction d'une combinaison desdites équations de mesure déterminées.
8. Dispositif selon l'une des clauses 6 et 7, caractérisé en ce que lesdites hypothèses sont transmises auxdits moyens de traitement par l'utilisateur effectuant les désignations.
9. Dispositif selon l'une des clauses 6 à 8, caractérisé en ce que lesdits moyens de traitement sont agencés pour déterminer lesdites hypothèses.
10. Dispositif selon l'une des clauses 1 à 9, caractérisé en ce que lesdits moyens de traitement sont agencés pour délivrer des données d'images représentatives d'un motif recalé de sorte que ledit motif puisse être observé en même temps que lesdites images d'observation et de façon superposée sur la portion d'images désignée correspondante.
11. Dispositif selon l'une des clauses 1 à 10, caractérisé en ce que ladite mémoire est propre à stocker une table de correspondance entre lesdits motifs tridimensionnels et des données d'information représentatives desdits motifs, et en ce que lesdits moyens de traitement sont agencés pour délivrer sur requête des données d'information représentatives d'un motif.
12. Dispositif selon la combinaison des clauses 10 et 11, caractérisé en ce que lesdits moyens de traitement sont agencés pour délivrer sensiblement simultanément sur requête des données d'images représentatives d'un motif recalé et les données d'information représentatives dudit motif recalé.
13. Installation de guidage par localisation, comprenant des moyens d'observation propres à délivrer des images d'une région choisie sous un angle choisi et un afficheur permettant d'afficher lesdites images, caractérisée en ce qu'elle comprend en outre une interface homme/machine agencée pour permettre à un utilisateur de désigner au moins une portion de la région représentée par lesdites images affichées et au moins un attribut représentatif de ladite portion, et un dispositif de recalage selon l'une des clauses précédentes, alimenté en portion et attribut désignés par ladite interface homme/machine et délivrant des données d'images représentatives d'un motif recalé de sorte que ledit motif soit superposé par ledit afficheur sur la portion désignée affichée.
14. Installation selon la clause 13, caractérisée en ce que ledit dispositif de recalage est agencé pour délivrer sur requête audit afficheur des données d'information représentatives d'un motif recalé sensiblement en même temps que les données d'images représentatives dudit motif recalé de sorte que ces données d'information soient superposées auxdites images d'observation affichées.
15. Installation selon l'une des clauses 13 et 14, caractérisée en ce que lesdits moyens d'observation comprennent des moyens d'acquisition endoscopique dont la position est connue à chaque instant par rapport à un référentiel de calibration, à partir duquel est définie la position de la région observée, et délivrant audit afficheur des images d'observation.
16. Installation selon l'une des clauses 13 à 15, caractérisée en ce que ledit afficheur comprend au moins un moniteur d'ordinateur.
17. Installation selon l'une des clauses 13 à 15, caractérisée en ce que ledit afficheur est agencé sous la forme de lunettes d'observation.
18. Installation selon l'une des clauses 13 à 17, caractérisée en ce qu'elle comprend des moyens de contrôle agencés, à réception d'une requête d'utilisateur désignant un motif de ladite région choisie, pour ordonner audit dispositif de recalage de déterminer des données de position représentatives de la position dudit motif désigné par rapport audit référentiel de calibration, compte tenu du recalage, puis pour déterminer des instructions de pilotage destinées à guider ledit utilisateur vers la portion de région correspondant audit motif désigné.
19. Installation selon l'une des clauses 13 à 18, caractérisée en ce qu'elle comprend un robot d'intervention comportant des bras, dont les positions respectives par rapport audit référentiel de calibration sont connues à chaque instant et propres à être pilotés à distance par des instructions fournies par un utilisateur via ladite interface homme/machine.
20. Installation selon la clause 19, caractérisée en ce qu'elle comprend des moyens de contrôle couplés audit dispositif de recalage et à ladite interface homme/machine, et agencés, à réception d'une requête d'utilisateur désignant un motif de ladite région choisie,
   i) pour ordonner audit dispositif de recalage de déterminer des données de position représentatives de la position dudit motif désigné par rapport audit référentiel de calibration compte tenu du recalage, et
   ii) pour déterminer des instructions de pilotage destinées à déplacer lesdits bras du robot au voisinage de la portion de région correspondant audit motif désigné.
21. Procédé de recalage en temps réel de motifs sur des images d'une région, caractérisé en ce qu'il consiste à:
   - stocker dans une mémoire des motifs représentatifs de portions d'une région choisie et de position et d'orientation connues par rapport à un référentiel commun,
   - observer sous un angle choisi ladite région choisie et délivrer en temps réel des images d'observation de ladite région,
   - désigner au moins une portion d'image d'observation de ladite région choisie et au moins un attribut représentatif de ladite portion,
   - déterminer dans ladite mémoire un motif représentatif de ladite portion désignée, compte tenu dudit attribut désigné, et
   - recaler ledit motif sur ladite portion d'image désignée compte tenu dudit angle choisi.
22. Procédé selon la clause 21, caractérisé en ce que l'on effectue ledit recalage par des désignations successives de portions d'image d'observation de ladite région choisie et/ou d'attributs représentatif desdites portions.
23. Procédé selon l'une des clauses 21 et 22, caractérisé en ce que lesdits motifs sont tridimensionnels.
24. Procédé selon la clause 23, caractérisé en ce que l'on stocke dans ladite mémoire un modèle tridimensionnel représentatif de ladite région au sein dudit référentiel choisi, et en ce que l'on effectue ledit recalage au moins à partir dudit modèle tridimensionnel stocké.
25. Procédé selon l'une des clauses 21 à 24, caractérisé en ce que l'on effectue un recalage de type rigide, par minimisation d'un critère choisi.
26. Procédé selon la clause 25, caractérisé en ce que l'on détermine au moins une équation de mesure à partir de la désignation d'une portion d'image, d'un attribut et d'au moins une hypothèse, et l'on procède à ladite détermination du motif et à son recalage rigide en fonction de l'équation de mesure déterminée.
27. Procédé selon la clause 25, caractérisé en ce que l'on détermine plusieurs équations de mesure à partir de la désignation de plusieurs portions d'images, d'attributs et d'hypothèses, et l'on procède à ladite détermination du motif et à son recalage rigide en fonction d'une combinaison desdites équations de mesure déterminées.
28. Procédé selon l'une des clauses 26 et 27, caractérisé en ce que certaines au moins desdites hypothèses sont transmises par l'utilisateur effectuant les désignations.
29. Procédé selon l'une des clauses 21 à 28, caractérisé en ce que l'on délivre des données d'images représentatives d'un motif recalé de sorte qu'il soit observé en même temps que lesdites images d'observation et de façon superposée sur la portion d'image désignée correspondante.
30. Procédé selon l'une des clauses 21 à 29, caractérisé en ce que l'on stocke dans ladite mémoire une table de correspondance entre lesdits motifs et des données d'information représentatives desdits motifs, et l'on délivre sur requête des données d'information représentatives d'un motif.
31. Procédé selon la combinaison des clauses 29 et 30, caractérisé en ce que l'on affiche lesdites données d'images représentatives d'un motif recalé.
32. Procédé selon l'une des clauses 21 à 31, caractérisé en ce que lesdites images d'observation sont délivrées par des moyens d'observation dont la position est connue à chaque instant par rapport à un référentiel de calibration, à partir duquel est définie la position de la région observée, et en ce que l'on procède auxdites désignations via une interface homme/machine.
33. Procédé selon l'une des clauses 21 à 32, caractérisé en ce que l'on délivre lesdites images d'observation sur au moins un moniteur d'ordinateur.
34. Procédé selon l'une des clauses 21 à 32, caractérisée en ce que l'on délivre lesdites images d'observation dans des lunettes d'observation.
35. Procédé selon l'une des clauses 21 à 34, caractérisé en ce qu'à réception d'une requête d'un utilisateur désignant un motif de ladite région choisie, on détermine des données de position représentatives de la position dudit motif désigné par rapport au référentiel de calibration, compte tenu du recalage, puis on détermine des instructions de pilotage destinées à guider ledit utilisateur vers la portion de région correspondant audit motif désigné.
36. Procédé selon la clause 35, caractérisé en ce que l'on prévoit un robot d'intervention comportant des bras dont les positions respectives par rapport audit référentiel de calibration sont connues à chaque instant et propres à être pilotés à distance par des instructions fournies par un utilisateur via ladite interface homme/machine, et en ce qu'à réception d'une requête d'utilisateur désignant un motif de ladite région choisie, on détermine des données de position représentatives de la position dudit motif désigné par rapport audit référentiel de calibration, compte tenu du recalage, puis on détermine des instructions de pilotage destinées à déplacer lesdits bras du robot au voisinage de la portion de région correspondant audit motif désigné.
37. Utilisation des dispositif de recalage, installation de guidage par localisation et procédé de recalage selon l'une des clauses précédentes, dans un domaine choisi parmi la chirurgie, et en particulier la chirurgie dite "mini-invasive" et la chirurgie dite "ouverte", et le guidage urbain.

## Revendications

1. Appareil comportant:
un robot chirurgical comportant au moins un bras ayant une position déterminée par rapport à un référentiel de calibration;
une interface homme/machine configurée pour recevoir des instructions fournies par un utilisateur pour piloter à distance la position de l'au moins un bras;
un dispositif de recalage configuré pour, à la réception d'une désignation par l'utilisateur d'un motif d'une région observée d'une image affichée, déterminer des données de position représentatives de la position du motif par rapport au référentiel de calibration ; et
un contrôleur couplé au dispositif de recalage et à l'interface homme/machine, le contrôleur étant configuré pour émettre, via l'interface homme/machine, des instructions de pilotage pour déplacer l'au moins un bras du robot au voisinage de la portion de la région observée correspondant au motif désigné.

2. Appareil selon la revendication 1, le dispositif de recalage étant de plus configuré pour, sur la base de la désignation du motif et de la désignation par l'utilisateur d'un attribut d'identité dudit motif, déterminer les données de position en recalant le motif sur un motif tridimensionnel, 3D, enregistré dans une mémoire couplée au dispositif de recalage, le motif 3D étant associé avec une identité correspondant à l'attribut d'identité.

3. Appareil selon la revendication 2, le motif 3D étant enregistré dans la mémoire correspondant à un modèle anatomique 3D d'un patient.

4. Appareil selon la revendication 1, comportant un processeur configuré pour superposer une représentation graphique dudit motif déterminé sur l'image affichée.

5. Appareil selon la revendication 2, la désignation de l'attribut d'identité comportant un nom d'un objet correspondant au ou dans le motif.

6. Appareil selon la revendication 1, l'image affichée étant reçue d'un endoscope, l'au moins un bras étant manœuvrable dans un champ de vue de l'endoscope.

7. Appareil selon la revendication 1, le dispositif de recalage étant en outre configuré pour déterminer les données de position en recevant successivement des désignations de motifs et des attributs d'identité correspondants desdits motifs.

8. Appareil selon la revendication 1, le dispositif de recalage étant en outre configuré pour déterminer les données de position en minimisant un critère construit à partir d'au moins une équation de mesure.

9. Appareil selon la revendication 8, l'au moins une équation de mesure étant obtenue à partir d'une hypothèse associée au motif.

10. Appareil selon la revendication 1, l'interface homme/machine comportant un système de reconnaissance vocal.

11. Procédé comportant les étapes consistant à :
recevoir des instructions fournies par un utilisateur pour piloter à distance une position d'au moins un bras d'un robot chirurgical, l'au moins un bras ayant une position déterminée par rapport à un référentiel de calibration ;
recevoir une désignation par l'utilisateur d'un motif d'une région observée d'une image affichée ;
à la réception de la désignation, déterminer des données de position représentatives de la position du motif par rapport au référentiel de calibration ; et
émettre pour l'utilisateur des instructions de pilotage pour déplacer l'au moins un bras de robot au voisinage de la région observée correspondant au motif désigné.

12. Procédé selon la revendication 11, comportant en outre une étape consistant à :
sur la base de la désignation du motif et de la désignation par l'utilisateur d'un attribut d'identité dudit motif, déterminer les données de position en recalant le motif sur un motif tridimensionnel, 3D, enregistré dans une mémoire couplée au dispositif de recalage, le motif 3D étant associé avec une identité correspondant à l'attribut d'identité.

13. Procédé selon la revendication 12, le motif 3D étant enregistré dans la mémoire correspondant à un modèle anatomique 3D d'un patient.

14. Procédé selon la revendication 11, comportant en outre une étape consistant à :
superposer une représentation graphique du motif déterminé sur l'image affichée.

15. Procédé selon la revendication 12, la désignation de l'attribut d'identité comportant un nom d'un objet correspondant au ou dans le motif.

16. Procédé selon la revendication 11, l'image affichée étant reçue d'un endoscope, l'au moins un bras étant manœuvrable dans un champ de vue de l'endoscope.

17. Procédé selon la revendication 11, comportant en outre une étape consistant à :
déterminer les données de position en recevant successivement des désignations de motifs et des attributs d'identité correspondants desdits motifs.

18. Procédé selon la revendication 11, comportant en outre une étape consistant à :
déterminer les données de position en minimisant un critère construit à partir d'au moins une équation de mesure.

19. Procédé selon la revendication 18, l'au moins une équation de mesure étant obtenue à partir d'une hypothèse associée au motif.

20. Procédé selon la revendication 11, l'interface homme/machine comportant un système de reconnaissance vocal.
